(19)

**Europäisches Patentamt**
**European Patent Office**
**Office européen des brevets**

(11) **EP 4 737 485 A1**

(12) **EUROPEAN PATENT APPLICATION**
published in accordance with Art. 153(4) EPC

(43) Date of publication:
06.05.2026 Bulletin 2026/19

(21) Application number: 24831593.9

(22) Date of filing: 05.06.2024

(51) International Patent Classification (IPC):
*C08B 15/02* (2006.01)    *A61K 8/73* (2006.01)
*C08B 15/04* (2006.01)    *C08L 1/04* (2006.01)
*C09K 3/00* (2006.01)

(52) Cooperative Patent Classification (CPC):
A61K 8/73; C08B 15/02; C08B 15/04; C08L 1/04;
C09K 3/00

(86) International application number:
PCT/JP2024/020495

(87) International publication number:
WO 2025/004723 (02.01.2025 Gazette 2025/01)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 30.06.2023  JP 2023107874
13.11.2023  JP 2023193250
13.11.2023  JP 2023193251

(71) Applicant: Toagosei Co., Ltd.
Minato-ku
Tokyo 105-8419 (JP)

(72) Inventors:
• TAKADA, Jun
Tokyo 105-8419 (JP)
• GOTOU, Akihiro
Tokyo 105-8419 (JP)
• SEKIGUCHI, Jueri
Tokyo 105-8419 (JP)

(74) Representative: dompatent
Partnerschaft von
Patentanwälten und Rechtsanwälten mbB
Deichmannhaus am Dom
Bahnhofsvorplatz 1
50667 Köln (DE)

(54) **CELLULOSE-CONTAINING COMPOSITION, METHOD FOR PRODUCING SAME, AND METHOD FOR ADJUSTING VISCOSITY**

(57)    A composition containing: a cellulose; and a thickener, in which the cellulose contains an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

[Fig.1]

EP 4 737 485 A1

## Description

Technical Field

The present invention relates to a cellulose-containing composition, a method for producing the same, and a viscosity modification method.

Background Art

Various technologies have been proposed with regard to producing nanocellulose materials by the oxidation of various types of cellulose raw-materials with an oxidant and the microfine-sizing of the resulting oxidized cellulose.

For example, Patent Literature 1 discloses a method for producing cellulose nanofiber having a step of oxidizing a cellulose raw-material with hypochlorous acid or a salt thereof having an available chlorine concentration of 14 to 43 mass% to produce oxidized cellulose and a step of fibrillating the oxidized cellulose into nanocellulose. Patent Literature 2 discloses a method for producing oxidized cellulose by subjecting a cellulose raw-material to an oxidation reaction using hypochlorous acid or a salt thereof having an available chlorine concentration of 6 mass% to 14 mass% while controlling pH to fall within a range of 5.0 to 14.0. In these methods, the oxidation is performed without using an N-oxyl compound such as 2,2,6,6-tetramethylpiperidin-1-oxyl (TEMPO), an N-oxyl radical, so that no N-oxyl compounds remain in the cellulose fiber. As a result, it is possible to reduce environmental impact and other associated effects.

Patent Literature 3 discloses oxidized cellulose that is an oxide obtained by oxidizing a cellulose raw-material with hypochlorous acid or a salt thereof, is substantially free of an N-oxyl compound, and has a degree of polymerization of 600 or less.

Patent Literature 4 discloses nanocellulose that is an oxide obtained by oxidizing a cellulose raw-material with hypochlorous acid or a salt thereof, has an average fiber width of 1 nm or more and 200 nm or less, is substantially free of an N-oxyl compound, and has a zeta potential of -30 mV or less.

Citation List

Patent Literatures

Patent Literature 1: WO 2018/230354 A
Patent Literature 2: WO 2020/027307 A
Patent Literature 3: WO 2022/009979 A
Patent Literature 4: WO 2022/009980 A

Summary of Invention

Technical Problem

An object of the present invention is to provide a composition containing an oxidized cellulose that is an oxide obtained by oxidizing a cellulose raw-material with a hypochlorous acid or a salt thereof and/or a nanocellulose that is a fibrillated product of the oxidized cellulose, and having a suitably modifiable (or modified) viscosity.

Solution to Problem

The present inventors and the like have found that in a case where a dispersion containing an oxidized cellulose that is an oxide obtained by oxidizing a cellulose raw-material with a hypochlorous acid or a salt thereof and/or a nanocellulose that is a fibrillated product of the oxidized cellulose is mixed with a thickener, the viscosity remarkably increases.

The present invention includes the following embodiments.

[1] A composition comprising: a cellulose; and a thickener, in which
the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.
[2] A composition comprising: a cellulose; and a thickener, in which

the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

[3] The composition according to [1] or [2], in which the thickener is a thickening polysaccharide.

[4] The composition according to any one of [1] to [3], in which the thickener has a hydrophobic region capable of hydrophobic interaction with the oxidized cellulose.

[5] The composition according to any one of [1] to [4], in which an amount of the thickener is 0.1 to 40 mass% with respect to a mass (solid content) of the oxidized cellulose.

[6] The composition according to any one of [1] to [5], further comprising a salt.

[7] The composition according to [6], in which an amount of the salt is 0.01 to 10 mass% with respect to the composition.

[8] The composition according to any one of [1] to [7], further comprising a pH adjuster.

[9] The composition according to [8], in which the composition has a pH in a range of 0 or more and 14.0 or less.

[10] The composition according to [8] or [9], in which the pH adjuster comprises an acid or a base.

[11] The composition according to any one of [1] to [10], in which the cellulose comprises the nanocellulose.

[12] The composition according to any one of [1] to [11], in which the composition is an aqueous composition.

[13] The composition according to [12], in which the aqueous composition has a viscosity of 0.01 to 1000 Pa·s.

[14] The composition according to [12] or [13], in which the aqueous composition has a viscosity of 10 to $1 \times 10^6$% higher than a viscosity of a control composition obtained by removing the thickener from the aqueous composition.

[15] A composition comprising: a cellulose; and a thickener, in which

the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound, and
the composition has a pH in a range of 0 or more and 14.0 or less.

[16] A composition comprising: a cellulose; and a thickener, in which

the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose, and
the composition has a pH in a range of 0 or more and 14.0 or less.

[17] The composition according to any one of [1] to [16], in which the composition is a cosmetic product.

[18] A method for producing a composition including:

a step of mixing a cellulose and a thickener, in which
the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

[19] A method for producing a composition including:

a step of mixing a cellulose and a thickener, in which
the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

[20] A method for modifying a viscosity of an aqueous composition comprising a cellulose, the method including:

a step of mixing the aqueous composition and a thickener, in which
the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

[21] A method for modifying a viscosity of an aqueous composition comprising a cellulose, the method including:

a step of mixing the aqueous composition and a thickener, in which

the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

[22] The method according to [20] or [21], in which the viscosity of the aqueous composition is increased by 10 to $1 \times 10^6$% by mixing the aqueous composition and the thickener.

Advantageous Effects of Invention

According to the present invention, it is possible to provide the composition containing an oxidized cellulose that is an oxide obtained by oxidizing a cellulose raw-material with a hypochlorous acid or a salt thereof and/or a nanocellulose that is a fibrillated product of the oxidized cellulose, and having a suitably modifiable (or modified) viscosity.

Brief Description of Drawings

[Figure 1]Fig. 1 shows the various viscosities of CNF, and the viscosity of a mixture of the CNF and methyl cellulose (10 mass% with respect to the mass of CNF).
[Figure 2]Fig. 2 shows the various viscosities of CNF, and the viscosity of a mixture of the CNF and methyl cellulose (2 mass% with respect to the mass of CNF).
[Figure 3]Fig. 3 shows the various viscosities of CNF, and the viscosity of a mixture of the CNF and methyl cellulose (10 mass% with respect to the mass of CNF).
[Figure 4]Fig. 4 shows the various viscosities of CNF, and the viscosity of a mixture of the CNF and methyl cellulose (2 mass% with respect to the mass of CNF).
[Figure 5]Fig. 5 shows the viscosity of an emulsion containing an acrylic resin, and the viscosity of a mixture of the emulsion and the CNF and/or methyl cellulose.
[Figure 6]Fig. 6 shows the viscosity of the CNF, the viscosity of a mixture of the CNF and methyl cellulose (1.4 mass%, 7.1 mass%, and 14 mass% with respect to the mass of CNF), and the viscosity of a mixture of CNF and guar gum (1.4 mass%, 7.1 mass%, and 14 mass% with respect to the mass of CNF).
[Figure 7]Fig. 7 shows the viscosities of compositions whose salt concentrations have been varied (Example 5 and Comparative Example 4).
[Figure 8]Fig. 8 shows the viscosities of compositions whose salt concentrations have been varied (Example 5 and Comparative Example 5).
[Figure 9]Fig. 9 shows the viscosity of a composition whose salt concentration has been varied (Example 5 and Comparative Example 6).
[Figure 10]Fig. 10 shows the viscosities of compositions whose salt concentrations have been varied (Example 5 and Comparative Example 7).
[Figure 11]Fig. 11 shows the relationship between pH and the viscosity of each composition of Example 6 and Comparative Examples 8 and 9.

Description of Embodiments

Hereinafter, embodiments of the present invention will be more specifically described, but the present invention is not limited thereto, and can be modified in various ways without departing from the gist thereof.

<Composition>

One embodiment of the present invention relates to a composition containing an oxidized cellulose that is an oxide obtained by oxidizing a cellulose raw-material with a hypochlorous acid or a salt thereof and/or a nanocellulose that is a fibrillated product of the oxidized cellulose, and a thickener.
A dispersion containing oxidized cellulose and/or nanocellulose in the present embodiment tends to have a lower viscosity than a dispersion containing oxidized cellulose and/or nanocellulose (hereinafter, also referred to as a "comparative cellulose") obtained by other methods (for example, TEMPO oxidation or mechanical fibrillation).
Surprisingly, the viscosity of the dispersion containing the comparative cellulose is less likely to increase even in a case where the comparative cellulose is mixed with the thickener, whereas the viscosity of the dispersion containing oxidized cellulose and/or nanocellulose in the present embodiment remarkably increases in a case where oxidized cellulose and/or nanocellulose is mixed with the thickener. Therefore, since the viscosity can be increased with a small amount of the thickener, the viscosity can be easily modified.

In addition, since the dispersion containing oxidized cellulose and/or nanocellulose in the present embodiment preferably exhibits thixotropic properties, the viscosity can temporarily decrease upon application of a force. As a result, for example, in a case where the dispersion containing oxidized cellulose and/or nanocellulose in the present embodiment is used as a coating material, a cosmetic product, or the like, the viscosity decreases during application, resulting in ease of use, and the viscosity increases after application, resulting in the facilitation of retention.

The reason why the viscosity is remarkably increased by mixing the oxidized cellulose and/or nanocellulose in the present embodiment with the thickener is presumed to be that an interaction (preferably a hydrophobic interaction) occurs between the oxidized cellulose and/or the nanocellulose, and the thickener, but the present invention is not limited to the presumption.

The pH of the composition according to the present embodiment is not particularly limited, but is preferably in a range of 0 or more and 14.0 or less from the viewpoint of minimizing a decrease in viscosity.

The lower limit value of the pH of the composition according to the present embodiment is preferably 2.0 and more preferably 2.5 from the viewpoint of retaining the viscosity.

The upper limit value of the pH of the composition according to the present embodiment is preferably 13.0 and more preferably 12.0 from the viewpoint of retaining the viscosity.

The pH of the composition according to the present embodiment is preferably 2.0 or more and 13.0 or less, and more preferably 2.5 or more and 12.0 or less.

The pH of the composition according to the present embodiment may be in a range of 0 or more and less than 7.0, in a range of 0 or more and 6.9 or less, in a range of 0 or more and 6.8 or less, in a range of 0 or more and 6.7 or less, in a range of 0 or more and 6.6 or less, in a range of 0 or more and 6.5 or less, in a range of more than 7.0 and 14.0 or less, in a range of 7.1 or more and 14.0 or less, in a range of 7.2 or more and 14.0 or less, in a range of 7.3 or more and 14.0 or less, in a range of 7.4 or more and 14.0 or less, or in a range of 7.5 or more and 14.0 or less.

The pH range of the composition according to the present embodiment may be a range obtained by appropriately combining the above-described ranges. Specific examples of the combined range include a range of 0 or more and less than 7.0 and more than 7.0 and 14.0 or less, and alternatively, a range of 0 or more and less than 7.0 and 7.5 or more and 14.0 or less.

The pH of the composition can be adjusted by, for example, adding a pH adjuster described later.

The pH of the composition according to the present embodiment can be measured by a pH meter.

The amount of the oxidized cellulose and/or nanocellulose in the present embodiment is not particularly limited, and is preferably 0.01 to 50 mass%, more preferably 0.1 to 30 mass%, still more preferably 0.1 to 20 mass%, and particularly preferably 0.1 to 15 mass% with respect to the mass of the composition. In a case where both oxidized cellulose and nanocellulose are contained in the composition, the above-described amount means the total amount of the oxidized cellulose and the nanocellulose.

The composition according to the present embodiment may not contain a dispersion medium. In other words, the composition according to the present embodiment may be in a dry state. The composition in a dry state is mixed with a dispersion medium, as necessary, and the viscosity thereof can be modified as appropriate.

The composition according to the present embodiment preferably contains a dispersion medium. The dispersion medium preferably contains water, and more preferably contains only water from the viewpoint of safety. In the present specification, the composition containing water as a dispersion medium is referred to as an "aqueous composition".

The viscosity of the aqueous composition according to the present embodiment may be appropriately modified according to the intended use. The viscosity of the aqueous composition may be, for example, 0.01 to 1000 Pa·s, 0.1 to 100 Pa·s, or 0.1 to 10 Pa·s.

In the present specification, unless otherwise specified, the term "viscosity" refers to a viscosity at a shear rate of $0.1\ s^{-1}$ when measured using a shear-type rheometer at 25°C and a shear rate range of 0.01 to 100 $s^{-1}$. A specific method for measuring the viscosity is as described in Examples described later.

The viscosity of the aqueous composition according to the present embodiment, as measured at a shear rate of 10 $s^{-1}$, is preferably 10 to $1 \times 10^6$% higher, more preferably 100 to $1 \times 10^5$% higher, and still more preferably $1 \times 10^3$ to $1 \times 10^5$% higher than the viscosity of a control composition obtained by removing the thickener from the aqueous composition. The viscosity increase rate is calculated by the following Equation.

Viscosity increase rate (%) = [(Viscosity of aqueous composition - Viscosity of control composition)/(Viscosity of control composition)] $\times$ 100

[Oxidized Cellulose]

The "oxidized cellulose" in the present embodiment refers to an oxide that is obtained by oxidizing a cellulose raw-material with hypochlorous acid or a salt thereof and is in a state prior to fibrillation.

Examples of hypochlorous acid or a salt thereof include aqueous hypochlorous acid, sodium hypochlorite, potassium hypochlorite, calcium hypochlorite, and ammonium hypochlorite.

The use amount of hypochlorous acid or a salt thereof is not particularly limited, and it is preferable to use hypochlorous acid or a salt thereof so that an available chlorine concentration in the reaction system is 6 to 43 mass%. The available chlorine concentration may be as low as 6 to 14 mass% or as high as 14 to 43 mass%.

The definition of the available chlorine concentration of hypochlorous acid or a salt thereof is as described in WO 2022/009979 A.

The cellulose raw-material is not particularly limited as long as it is a material mainly containing cellulose. Examples thereof include pulp, natural cellulose, and microfibrillated cellulose depolymerized by mechanical treatment of cellulose. The cellulose raw-material preferably has type-I crystal structure. As the cellulose raw-material, a commercially available product of crystalline cellulose or the like using pulp as a raw material can be directly used. Other than this, unutilized biomass rich in cellulose component, such as okara or soy hull, may be used as a raw material. For facilitating permeation of the oxidant to be used into the starting pulp, the cellulose raw-material may be previously treated with an alkali having an appropriate concentration.

Note that cellulose is a main component of plants, and a bundle of cellulose molecules is referred to as a cellulose microfibril. The cellulose in the cellulose raw-material is also contained in the form of cellulose microfibril.

(N-Oxyl Compound)

The oxidized cellulose in the present embodiment is substantially free of an N-oxyl compound. Since the oxidized cellulose is substantially free of an N-oxyl compound, the influences on the environment and human bodies are sufficiently reduced, and the high safety is achieved. Examples of the N-oxyl compound include 2,2,6,6-tetramethylpiperidin-1-oxyl (TEMPO).

In the present specification, the phrase "substantially free of an N-oxyl compound" means that an N-oxyl compound is not used in producing oxidized cellulose; an N-oxyl compound is not contained at all in oxidized cellulose; or the content of the N-oxyl compound is 2.0 mass ppm or less, and preferably 1.0 mass ppm or less, with respect to the total amount of oxidized cellulose.

A case where the content of an N-oxyl compound, as an increase derived from the cellulose raw-material, is preferably 2.0 mass ppm or less and more preferably 1.0 mass ppm or less is also regarded as "substantially free of an N-oxyl compound."

The content of an N-oxyl compound can be determined by commonly known means. Examples of the commonly known means include a method using a total trace nitrogen analyzer (for example, TN-2100H manufactured by Nittoseiko Analytech Co., Ltd.).

(Carboxy Group Content)

The carboxy group content of the oxidized cellulose in the present embodiment is preferably 0.1 to 3.0 mmol/g, more preferably 0.2 to 2.0 mmol/g, still more preferably 0.3 to 1.5 mmol/g, particularly preferably 0.4 to 1.2 mmol/g, and most preferably 0.5 to 0.9 mmol/g.

The carboxy group content of the oxidized cellulose can be measured by a method described in WO 2022/009979 A.

The oxidized cellulose of the present embodiment preferably has a structure in which at least two of the hydroxyl groups of a glucopyranose ring constituting cellulose are oxidized, and more specifically, a structure in which the hydroxyl groups at position 2 and position 3 of the glucopyranose ring are oxidized and dicarboxy groups are introduced. It is preferable that the hydroxyl group at position 6 of the glucopyranose ring is not oxidized and remains as it is. Note that the positions of the carboxy groups in the glucopyranose ring can be analyzed using solid-state $^{13}$C-NMR spectroscopy.

Rayon has the same chemical structure as cellulose, and an oxide thereof (oxidized rayon) is soluble in water. In a case where oxidized rayon is dissolved in heavy water and solution-state one-dimensional $^{13}$C-NMR measurement is performed, a peak of carbon belonging to the carboxy group is observed at 165 to 185 ppm. In one aspect of an oxide obtained by oxidizing a cellulose raw-material with hypochlorous acid or a salt thereof, two signals appear in this chemical-shift range. When solution-state two-dimensional NMR measurement is further performed, it can be determined that the carboxy groups are introduced into position 2 and position 3.

In solid-state $^{13}$C-NMR of a cellulose raw-material oxidized with hypochlorous acid or a salt thereof, two signals appear at 165 to 185 ppm when a carboxy group is introduced in a large amount, whereas when a carboxy group is introduced in a small amount, a very broad signal can appear. As is apparent from the results of oxidized rayon, the signals of carbons of the carboxy groups introduced at the position 2 and position 3 are close to each other, and separation between the two signals is insufficient in low-resolution solid-state $^{13}$C-NMR. As described above, in a case where the introduction amount of the carboxy group is low, a broad signal is observed. In other words, in the solid-state $^{13}$C-NMR

spectrum, it can be confirmed that carboxy groups are introduced at positions 2 and 3 based on evaluation of the broad peak appearing at 165 to 185 ppm.

That is, a baseline is added to the peak in the range of 165 ppm to 185 ppm in the solid-state $^{13}$C-NMR spectrum and the overall area value is obtained. Thereafter, the ratio of the two peak area values (larger area value/smaller area value), which is obtained by the perpendicular partitioning of the area value at the peak top, is determined. In a case where the ratio of the peak area values is 1.2 or more, the peak can be determined as a broad peak.

The presence/absence of the broad peak can be determined based on the ratio between the length L of the baseline in the range of 165 ppm to 185 ppm and the length L' of the perpendicular line from the peak top to the baseline. Thus, when the ratio L'/L is 0.1 or more, it can be determined that the broad peak is present. This ratio L'/L may be 0.2 or more, 0.3 or more, 0.4 or more, or 0.5 or more. The upper limit value of the ratio L'/L is not particularly limited and may be generally 3.0 or less, 2.0 or less, or 1.0 or less.

The structure of the above-described glucopyranose ring can be determined also by analysis based on a method described in Sustainable Chem. Eng. 2020, 8, 48, 17800-17806.

(Viscosity-Average Degree of Polymerization)

The viscosity-average degree of polymerization of the oxidized cellulose in the present embodiment is preferably 30 to 500, more preferably 60 to 300, still more preferably 70 to 150, and particularly preferably 80 to 130.

The viscosity-average degree of polymerization refers to an average degree of polymerization determined by the viscosity method. The viscosity-average degree of polymerization can be determined by a method described in WO 2022/009979 A.

[Method for Producing Oxidized Cellulose]

The oxidized cellulose according to the present embodiment can be produced by oxidizing a cellulose raw-material with hypochlorous acid or a salt thereof. A production method is more specifically described in WO 2022/009979 A and WO 2022/009980 A. An oxidized cellulose may be a commercially available product, and examples thereof include ARONFIBRO (registered trademark) manufactured by Toagosei Co., Ltd.

[Nanocellulose]

The "nanocellulose" in the present embodiment refers to an oxide that is obtained by oxidizing a cellulose raw-material with hypochlorous acid or a salt thereof and is in a state subsequent to fibrillation.

Nanocellulose is a collective term meaning a microfibrillated cellulose material including microfibrillated cellulose fiber and cellulose nanocrystal (CNC). The microfibrillated cellulose fiber is also referred to as cellulose nanofiber (CNF). From the viewpoint of increasing the viscosity, the nanocellulose is preferably CNF. The nanocellulose preferably has an amorphous region. CNF has an amorphous region, whereas CNC does not have an amorphous region.

The nanocellulose in the present embodiment contains a carboxy group. The carboxy group may be in the protonated form (-COOH) or in the salt form. Examples of the salt include, but are not particularly limited to, alkali metal salts such as a lithium salt, a sodium salt, and a potassium salt; alkaline earth metal salts such as a calcium salt and a barium salt; other metal salts such as a magnesium salt and an aluminum salt; ammonium salts; and organic amine salts.

Nanocellulose refers to an aggregate of fibers on a single-fiber basis. In a case where the nanocellulose contains carboxylated nanocellulose, it is sufficient that the nanocellulose contains at least one carboxylated nanocellulose, and preferably contains carboxylated nanocellulose as a main component. Here, the phrase "contains carboxylated nanocellulose as a main component" means that the ratio of carboxylated nanocellulose relative to the total amount of nanocellulose is more than 50 mass%, preferably more than 70 mass%, and more preferably more than 80 mass%. The upper limit of the ratio is 100 mass%, and may be 98 mass% or 95 mass%.

(N-Oxyl Compound)

The nanocellulose in the present embodiment is substantially free of an N-oxyl compound. The meaning that nanocellulose "substantially free of an N-oxyl compound" and the method for measuring the content of an N-oxyl compound are the same as described in the section of (N-Oxyl Compound) in [Oxidized Cellulose] described above.

(Carboxy Group Content)

The carboxy group content of nanocellulose and the measurement method thereof in the present embodiment are the same as described in the section of (Carboxy Group Content) in [Oxidized Cellulose].

(Average Fiber Length)

The average fiber length of nanocellulose in the present embodiment is preferably 50 to 700 nm, more preferably 50 to 500 nm, still more preferably 50 to 300 nm, even still more preferably 60 to 300 nm, and particularly preferably 70 to 200 nm.

(Average Fiber Width)

The average fiber width of nanocellulose in the present embodiment is preferably 1 to 200 nm, more preferably 1 to 15 nm, still more preferably 1 to 10 nm, and particularly preferably 1 to 5 nm.
The average fiber width and average fiber length of nanocellulose can be measured by a method described in WO 2022/009980 A.

(Aspect Ratio)

The aspect ratio (average fiber length/average fiber width) of nanocellulose in the present embodiment is preferably 20 to 200, more preferably 30 to 190, and still more preferably 40 to 180.

(Zeta Potential)

The zeta potential of nanocellulose in the present embodiment is preferably -30 mV or less, more preferably - 90 mV or more and -30 mV or less, still more preferably -80 mV or more and -30 mV or less, even still more preferably - 70 mV or more and -30 mV or less, and particularly preferably -65 mV or more and -35 mV or less.
The zeta potential can be measured by a method described in WO 2022/009980 A.

(Light Transmittance)

A nanocellulose dispersion in which nanocellulose in the present embodiment is dispersed in a dispersion medium has less light scattering or the like of cellulose fiber, and thus can exhibit high light transmittance. Specifically, the light transmittance in a mixed solution obtained by mixing nanocellulose with water to have a solid content concentration of 0.1 mass% is preferably 95% or more, more preferably 96% or more, still more preferably 97% or more, and particularly preferably 99% or more. The light transmittance is the value measured at a wavelength of 660 nm by a spectrophotometer.
The light transmittance can be measured by a method described in WO 2022/009979 A.

[Method for Producing Nanocellulose]

The nanocellulose in the present embodiment can be produced by fibrillating the oxidized cellulose described above. A production method is more specifically described in WO 2022/009979 A and WO 2022/009980 A. The nanocellulose can also be obtained by fibrillating a commercially available oxidized cellulose (for example, ARONFIBRO (registered trademark) manufactured by Toagosei Co., Ltd., and the like).

[Thickener]

The composition according to the present embodiment contains a thickener. The viscosity can be significantly increased by combining the oxidized cellulose and/or nanocellulose in the present embodiment with the thickener. The thickener may be used singly or in combination of two or more kinds thereof.
The thickener is preferably a thickening polysaccharide. The thickening polysaccharide is preferably water-soluble. In the present specification, the term "water-soluble" means that the amount dissolved in 100 g of water at 20°C is 1 g or more. The dissolved amount is preferably 3 g or more, more preferably 10 g or more, and still more preferably 20 g or more.
Examples of the thickening polysaccharide include methyl cellulose, carboxymethyl cellulose, guar gum, xanthan gum, locust bean gum, cassia gum, gellan gum, psyllium seed gum, tragacanth gum, karaya gum, gum arabic, ghatti gum, tara gum, tamarind seed gum, carrageenan, pectin, pullulan, curdlan, starch, gelatin, agar, alginic acid, and soybean polysaccharides.
The thickener may be a thickener having a hydrophobic region capable of hydrophobic interaction with the oxidized cellulose and/or nanocellulose in the present embodiment (hereinafter, referred to as a "hydrophobic thickener").
Examples of the hydrophobic region of the hydrophobic thickener include a continuous cyclic structure and a linear or branched hydrocarbon chain structure.

The hydrophobic thickener having a continuous cyclic structure is obtained by, for example, polymerizing a monomer having a cyclic structure. Examples of the cyclic structure include an aromatic hydrocarbon structure and an alicyclic hydrocarbon structure. The cyclic structure is preferably a cycloalkane structure, and more preferably a cyclohexane structure. The thickening polysaccharide is generally included in the hydrophobic thickener because the thickening polysaccharide has a continuous cyclohexane structure.

The amount of the thickener may be appropriately adjusted according to a desired viscosity, and is preferably 0.1 to 40 mass%, more preferably 0.3 to 35 mass%, still more preferably 0.5 to 30 mass%, and particularly preferably 0.5 to 20 mass% with respect to the mass (solid content) of the oxidized cellulose and/or nanocellulose in the present embodiment. In a case where both oxidized cellulose and nanocellulose are contained in the composition, the "mass (solid content) of the oxidized cellulose and/or nanocellulose" means the "total mass (solid content) of the oxidized cellulose and nanocellulose". The dispersion containing the oxidized cellulose and nanocellulose in the present embodiment can significantly increase the viscosity with a small amount of the thickener.

[Salt]

The composition according to the present embodiment may further contain a salt. In the present specification, the salt is a compound composed of a cation (hereinafter, also referred to as a "cation") and an anion (hereinafter, also referred to as an "anion"). The viscosity increased by the combination of the oxidized cellulose and/or nanocellulose in the present embodiment and the thickener can be retained without decreasing even though a salt is contained.

As described above, it is presumed that the mechanism of the viscosity increase is because an interaction (preferably a hydrophobic interaction) occurs between the oxidized cellulose and/or nanocellulose and the thickener, and it is presumed that the viscosity decrease is minimized even in the presence of a salt because this interaction is less likely to be affected by the salt.

The salt may be used singly or in combination of two or more kinds thereof.

The salt may be an inorganic salt, an organic salt, or a mixture thereof.

Examples of a cation constituting an inorganic salt or an organic salt include alkali metal ions such as lithium ion ($Li^+$), sodium ion ($Na^+$), and potassium ion ($K^+$); alkaline earth metal ions such as magnesium ions ($Mg^{2+}$) and calcium ions ($Ca^{2+}$); transition metal ions such as iron ions ($Fe^{3+}$ and $Fe^{2+}$) and copper ions ($Cu^{2+}$ and $Cu^+$); and ions of Group 12 or Group 13 metals, such as zinc ions ($Zn^{2+}$) and aluminum ions ($Al^{3+}$). Among these cations, a monovalent cation is preferably used from the viewpoint of retaining the viscosity.

Examples of an anion constituting an inorganic salt or an organic salt include halogen ions such as chloride ions ($Cl^-$), bromide ions ($Br^-$), and iodide ions ($I^-$); sulfate ions ($SO_4^{2-}$); nitrate ions ($NO_3^-$); carbonate ions ($CO_3^{2-}$); bicarbonate ions ($HCO_3^-$); hydrogen sulfate ions ($HSO_4^-$); phosphate ions ($PO_4^{3-}$); hydrogen phosphate ions ($HPO_4^{2-}$); dihydrogen phosphate ions ($H_2PO_4^-$); sulfite ions ($SO_3^{2-}$); thiosulfate ions ($S_2O_3^{2-}$); chlorate ions ($ClO_3^-$); perchlorate ions ($ClO_4^-$); and ions having a group derived from an acidic group, such as a carboxy group (-C(=O)-OH) or a sulfo group ($-S(=O)_2-OH$), from which a hydrogen ion ($H^+$) has been removed.

Examples of the inorganic salt include chlorides of alkali metals such as sodium chloride (NaCl) and potassium chloride (KCl); hydrochlorides such as ammonium chloride ($NH_4Cl$); sulfates such as sodium sulfate ($Na_2SO_4$), potassium sulfate ($K_2SO_4$), ammonium sulfate (($NH_4)_2SO_4$), magnesium sulfate ($MgSO_4$), aluminum sulfate ($Al_2(SO_4)_3$), nickel sulfate ($NiSO_4$), alum ($AlK(SO_4)_2$), and ammonium alum ($Al(NH_4)(SO_4)_2$); nitrates such as sodium nitrate ($NaNO_3$), potassium nitrate ($KNO_3$), ammonium nitrate ($NH_4NO_3$), calcium nitrate tetrahydrate ($Ca(NO_3)_2 \cdot 4H_2O$), and calcium nitrate ($Ca(NO_3)$); carbonates such as potassium carbonate ($K_2CO_3$); bicarbonates such as potassium bicarbonate ($KHCO_3$) and sodium bicarbonate ($NaHCO_3$); chlorides of alkaline earth metals such as calcium chloride ($CaCl_2$) and magnesium chloride ($MgCl_2$); hydrogen phosphates such as sodium dihydrogen phosphate ($NaH_2PO_4$), disodium hydrogen phosphate ($Na_2HPO_4$), potassium dihydrogen phosphate ($KH_2PO_4$), dipotassium hydrogen phosphate ($K_2HPO_4$), ammonium dihydrogen phosphate ($NH_4H_2PO_4$), and diammonium hydrogen phosphate (($NH_4)_2HPO_4$); phosphates such as trisodium phosphate ($Na_3PO_4$); sulfites such as sodium sulfite ($Na_2SO_4$); chlorates such as potassium chlorate ($KClO_3$); perchlorates such as sodium perchlorate ($NaClO_4$); thiosulfates such as sodium thiosulfate ($Na_2S_2O_3$); alkali metal bromides such as potassium bromide (KBr) and sodium bromide (NaBr); alkali metal iodides such as potassium iodide (KI) and sodium iodide (NaI); and borates such as borax ($Na_2B_4O_7$).

These inorganic salts may be used singly or in combination of two or more kinds thereof.

Among these cations, an inorganic salt having a monovalent cation is preferably used from the viewpoint of retaining the viscosity.

Examples of the organic salt include acetate, lactate, maleate, fumarate, tartrate, methanesulfonate, p-toluenesulfonate, triethanolamine salts, and amino acid salts.

The amount of the salt is preferably 0.01 to 10 mass%, more preferably 0.01 to 5 mass%, and still more preferably 0.05 to 5 mass% with respect to the mass of the composition.

The amount of the salt is preferably 1 to 1000 mass%, more preferably 1 to 500 mass%, and still more preferably 5 to 500 mass% with respect to the mass (solid content) of the oxidized cellulose and/or nanocellulose in the present embodiment. In a case where both oxidized cellulose and nanocellulose are contained in the composition, the "mass (solid content) of the oxidized cellulose and/or nanocellulose" means the "total mass (solid content) of the oxidized cellulose and nanocellulose".

[pH Adjuster]

The composition according to the present embodiment may further contain a pH adjuster. In the present specification, the pH adjuster is an agent having the function of adjusting the pH to a predetermined range The viscosity increased by the combination of the oxidized cellulose and/or nanocellulose in the present embodiment and the thickener can be retained without decreasing even though a pH adjuster is contained.

As described above, it is presumed that the mechanism of the viscosity increase is because an interaction (preferably a hydrophobic interaction) occurs between the oxidized cellulose and/or nanocellulose and the thickener, and it is presumed that the viscosity decrease is minimized even when the pH changes because this interaction is less likely to be affected by the pH.

The pH adjuster may be used singly or in combination of two or more kinds thereof.

The pH adjuster may be any of an acid, a base, and a neutralizing agent, but preferably includes an acid or a base. As the pH adjuster, an acid and a neutralizing agent may be used in combination, or a base and a neutralizing agent may be used in combination.

The acid may be an organic acid, an inorganic acid, or a mixture thereof.

Examples of the organic acid include citric acid, tartaric acid, fumaric acid, malic acid, maleic acid, gluconic acid, succinic acid, ascorbic acid, glycolic acid, and salicylic acid.

Examples of the inorganic acid include hydrochloric acid, sulfuric acid, phosphoric acid, and sulfamic acid.

The base may be an organic base, an inorganic base, or a mixture thereof.

Examples of the organic base include triethanolamine, carbamide peroxide, and cement phosphate.

Examples of the inorganic base include ammonia, ammonium hydroxide, and alkali metal salts such as lithium, sodium, and potassium.

The neutralizing agent is not particularly limited as long as it has the function of adjusting the pH to a target pH. The neutralizing agent can be used to adjust or maintain acidic or basic levels. The neutralizing agent can also be represented as a buffer. Examples of the neutralizing agent include citric acid, sodium carbonate, phosphoric acid, ammonium hydroxide, and glycolic acid.

The amount of the pH adjuster may be appropriately changed according to the target pH. The pH of the composition according to the present embodiment is as described above.

[Optional Component]

The composition according to the present embodiment may contain additional components (optional components). Even though an optional component is contained, a thickening effect can be exhibited.

The optional component may be appropriately selected according to the intended use, and examples thereof include a resin, a pigment, a surfactant, an antifoaming agent, a preservative, a plasticizer, a stabilizer, and an antioxidant.

[Cosmetic Product]

Examples of the aspect of the composition of the present embodiment include a coating material and a cosmetic product, and a cosmetic product is preferable.

Examples of the cosmetic product include makeup cosmetic products, hair cosmetic products, skin cosmetic products, perfumes, eau de colognes, bath preparations, nail makeup preparations, lip care preparations, and body powders.

Examples of the makeup cosmetic products include lip makeup preparations such as lipsticks and lip glosses; point makeup preparations such as eyeshadows, eyeliners, blushers, and mascara liquids; and base makeup preparations such as foundations, concealers, and powders.

Examples of the hair cosmetic products include hair styling preparations, hair coloring preparations (hair dyes), hair cleansing preparations, and rinses.

Examples of the skin cosmetic products include skin lotions, skin emulsions, creams, milky lotions, sun tanning preparations, sunscreen preparations, aftercare lotions, cleansing preparations, and facial masks.

Examples of the nail makeup preparations include manicure preparations, pedicure preparations, and nail polish removers.

The cosmetic product in the present specification also includes quasi-drugs. That is, those containing active ingredients intended for purposes such as prevention or hygiene are also included in the cosmetic products in the present specification.

Furthermore, for example, hair coloring preparations (hair dyes) may fall under either cosmetic products or quasi-drugs. Examples of the hair coloring preparations (hair dyes) include aspects of: permanent hair dyes such as hair color and gray hair dyes; bleaching agents such as hair bleaches; semi-permanent hair dyes such as hair manicures, color treatments, and color rinses; and temporary hair dyes such as hair mascaras, hair color sprays, hair markers, and hair foundations, and the hair coloring preparations (hair dyes) in the present specification include these aspects.

Another embodiment of the present invention relates to a composition containing a cellulose, and a thickener, in which the cellulose contains an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose. For the details (such as preferred properties and components) of the composition according to the present embodiment, the description of the composition according to the above-described embodiment are appropriately incorporated by reference.

<Method for Producing Composition>

An embodiment of the present invention relates to a method for producing a composition including a step of mixing a cellulose and a thickener, and as necessary, a salt and/or a pH adjuster, in which the cellulose contains an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

The details of the production method according to the present embodiment (for example, details of the composition and the materials thereof) follow the description in the above-described section <Composition>.

Since the oxidized cellulose obtained through an oxidation reaction with hypochlorous acid or a salt thereof is readily fibrillated, at least a portion of the oxidized cellulose is fibrillated into nanocellulose even by gentle mixing with the thickener.

Another embodiment of the present invention relates to a method for producing a composition including: a step of mixing a cellulose and a thickener, in which the cellulose contains an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose. For the details of the production method according to the present embodiment, the description of the production method according to the above-described embodiment are appropriately incorporated by reference.

<Method for Modifying Viscosity of Composition>

An embodiment of the present invention relates to a method for modifying a viscosity of an aqueous composition containing a cellulose including a step of mixing the aqueous composition and a thickener, and as necessary, a salt and/or a pH adjuster, in which the cellulose contains an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

The details of the method according to the present embodiment (for example, details of the aqueous composition and the materials thereof) follow the description in the above-described section <Composition>.

It is preferable that the viscosity of the aqueous composition, as measured at a shear rate of $10 \text{ s}^{-1}$ by mixing the aqueous composition and a thickener, is increased by 10% to $1 \times 10^{6}$%, more preferably by 100% to $1 \times 10^{5}$%, and still more preferably by $1 \times 10^{3}$% to $1 \times 10^{5}$%.

Another embodiment of the present invention relates to a method for modifying a viscosity of an aqueous composition containing a cellulose, the method including a step of mixing the aqueous composition and a thickener, in which the cellulose contains an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose. For the details of the modification method according to the present embodiment, the description of the modification method according to the above-described embodiment are appropriately incorporated by reference.

Examples

Hereinafter, the present invention will be described in more detail with reference to Examples and Comparative Examples, but the technical scope of the present invention is not limited thereto.

Note that various values in Examples may be preferable lower limit values or upper limit values in the embodiment of the present invention. In addition, two values of the same kind in Examples may be appropriately combined to form a preferable numerical range.

[Production Example 1]

As a cellulose raw-material, commercially available powdered cellulose was used.

350 g of sodium hypochlorite pentahydrate crystals, which had an available chlorine concentration of 42 mass%, was introduced into a beaker and pure water was added and stirring was performed to obtain an aqueous sodium hypochlorite solution having an available chlorine concentration of 21 mass%. To this was added 35 mass% hydrochloric acid with stirring to provide an aqueous solution having a pH of 11.0. This aqueous sodium hypochlorite solution was heated to 30°C using a thermostatted water bath while being stirred at 200 rpm with a propeller-form stirring blade using a stirrer (three-One Motor, BL600) manufactured by Shinto Scientific Co., Ltd., followed by addition of 50 g of the above-described powdered cellulose.

After the introduction of the cellulose raw-material, 48 mass% sodium hydroxide was added while the temperature was held at 30°C in the same thermostatted water tank, and the pH during the reaction was adjusted to 11.0; the oxidation reaction was then carried out by stirring at 200 rpm for 30 minutes with a propeller-form stirring blade using the above-described stirrer. After the completion of the reaction, the product was subjected to solid-liquid separation by suction filtration using a PTFE membrane filter having a pore size of 0.1 $\mu$m to obtain oxidized cellulose. The obtained oxidized cellulose was washed with pure water, and the carboxy group content of the residue (oxidized cellulose) measured after washing was 0.70 mmol/g.

In addition, an N-oxyl compound-derived nitrogen component in the oxidized cellulose was measured as the amount of nitrogen using a total trace nitrogen analyzer (manufactured by Nittoseiko Analytech Co., instrument name: TN-2100H); an increase derived from the starting pulp was calculated, giving a result of 1 ppm or less.

The available chlorine concentration in the aqueous sodium hypochlorite solution was measured by the following method.

(Measurement of Available Chlorine Concentration in Aqueous Sodium Hypochlorite Solution)

0.582 g of an aqueous solution of sodium hypochlorite pentahydrate crystals added to pure water was precisely weighed out, and 50 mL of pure water was added and 2 g of potassium iodide and 10 mL of acetic acid were added, followed immediately by tight sealing and standing for 15 minutes in a dark location. After the standing for 15 minutes, the liberated iodine was titrated with a 0.1 mol/L sodium thiosulfate solution, which resulted in a titration volume of 34.55 mL (indicator: starch reagent solution). Correction was performed by carrying out a separate blank test. Since 1 mL of the 0.1 mol/L sodium thiosulfate solution corresponded to 3.545 mg Cl, the available chlorine concentration in the aqueous sodium hypochlorite solution was 21 mass%.

The carboxy group content in the oxidized cellulose was measured using the following method.

(Measurement of Carboxy Group Content)

To 60 ml of aqueous oxidized cellulose dispersion controlled so as to have an oxidized cellulose concentration of 0.5 mass%, a 0.1 M aqueous hydrochloric acid solution was added to set pH at 2.5; a 0.05 N aqueous sodium hydroxide solution was then added dropwise; the electrical conductivity was measured until the pH reached 11.0; the amount (a) of sodium hydroxide, which was consumed in a stage of neutralizing weak acid where the electrical conductivity slightly changed, was obtained and substituted into the following equation; thereby, the carboxy group content (mmol/g) was computationally obtained.

Carboxy-group content = a (ml) $\times$ 0.05/mass (g) of oxidized cellulose

A sample was prepared by freeze-drying the oxidized cellulose obtained in Production Example 1 and then left to stand for 24 hours or longer at 23°C and 50% RH, and the solid-state $^{13}$C-NMR of the sample was measured. As a result, in all instances it was confirmed that a structure was present in which the hydroxyl groups at positions 2 and 3 of the glucopyranose ring were oxidized with the introduction of carboxy groups. The solid-state $^{13}$C-NMR measurement conditions are given below.

(1) Sample tube: zirconia tube (4 mm diameter)
(2) Magnetic field strength: 9.4 T (1H resonance frequency: 400 MHz)
(3) MAS rotation rate: 15 kHz
(4) Pulse sequence: CPMAS method

(5) Contact time: 3 ms

(6) Waiting time: 5 s

(7) Number of scans: 10,000 to 15,000

(8) Measurement instrument: JNM ECA-400 (JEOL Ltd.)

From the results obtained using a model molecule for the oxidized cellulose as the sample and carrying out measurement using two-dimensional NMR, it was also confirmed that the obtained oxidized cellulose had a structure in which the hydroxyl groups at positions 2 and 3 of the glucopyranose ring were oxidized with the introduction of carboxy groups.

With regard to position 6, no change in the spectroscopic data was seen between the solid-state $^{13}$C-NMR of the cellulose raw-material and the solid-state $^{13}$C-NMR of the oxidized cellulose, and based on this it was concluded that the hydroxyl group at position 6 was not oxidized and the hydroxyl group remained intact in the oxidized cellulose.

[Comparative Production Example 1]

The nanocellulose obtained by TEMPO oxidation was produced in accordance with Angew. Chem. Int. Ed. 2021, 60, 24630-24636. That is, the production method was as follows.

Into a beaker, 0.016 g TEMPO and 0.1 g sodium bromide were introduced; pure water was added with stirring to prepare an aqueous solution; and 1.0 g of powdered pulp (KC FLOCK W-100GK) manufactured by Nippon Paper Industries Co., Ltd. was added as a cellulose raw-material.

This aqueous solution was heated to 25°C in a thermostatted water bath while stirring with a stirrer, after which 0.1 mol/L sodium hydroxide was added with stirring to prepare an aqueous solution having a pH of 10.0. To this was added 2.58 g of an aqueous sodium hypochlorite solution having an available chlorine concentration of 13.2 mass%, and while the temperature was held at 25°C in the same thermostatted water tank, stirring with a stirrer was performed for 120 minutes with the pH adjusted to 10.0 during the reaction by the addition of 0.1 mol/L sodium hydroxide.

After the completion of the reaction, the product was subjected to solid-liquid separation by suction filtration using a PTFE membrane filter having a pore size of 0.1 $\mu$m to obtain oxidized cellulose. The obtained residue was washed with pure water, followed by measurement of the carboxy group content. The carboxy group content was 1.55 mmol/g, and the amount of the residue was approximately 1.0 g. In addition, the N-oxyl compound-derived nitrogen component in the oxidized cellulose was measured as the amount of nitrogen using a total trace nitrogen analyzer (manufactured by Nittoseiko Analytech Co., instrument name: TN-2100H); an increase derived from the starting pulp was calculated, giving a result of 5 ppm. By mechanically fibrillating this oxidized cellulose, an aqueous nanocellulose dispersion (solid content of 2.0 mass%) obtained by TEMPO oxidation was obtained.

[Comparative Production Example 2]

An aqueous nanocellulose dispersion (solid content of 2.0 mass%) was obtained by mechanically fibrillating commercially available powdered cellulose using a Starburst system.

[Example 1]

The oxidized cellulose obtained in Production Example 1 was used as an aqueous dispersion (solid content of about 10 mass%), and distilled water was added thereto to dilute the oxidized cellulose to a solid content of 5 mass%. In this state, the mixture was stirred at 10000 rpm for 60 minutes using a homomixer (TOKUSHU KIKA ROBO MICS) to fibrillate the oxidized cellulose into CNF.

Separately, an aqueous solution having a concentration of 1 mass% of methyl cellulose (MC) and distilled water were prepared. A CNF aqueous dispersion having a solid content of 5 mass%, a 1 mass% aqueous solution of methyl cellulose, and distilled water were mixed to prepare a sample having a predetermined ratio of CNF/methyl cellulose/water (see the results for specific ratios).

The viscosity measurement method of each sample was as follows.

A shear-type rheometer (Anton Paar, Physica MCR301) was used for viscosity measurement, and a parallel disk type having a diameter of 50 mm was used as a jig. The shear rate was set to 0.01 to 100 s$^{-1}$ and the temperature was set to 25°C. The measurement was performed twice for one sample (measurement at 0.01 to 100 s$^{-1}$ was performed once, and the sample was left to stand for 20 seconds after the end of the measurement. Thereafter, a second measurement was performed in the same shear rate range), and the second data was adopted.

[Comparative Example 1]

The aqueous nanocellulose dispersion (solid content of 2.0 mass%) obtained by TEMPO oxidation, which was produced in Comparative Production Example 1, a 1 mass% aqueous solution of methyl cellulose, and distilled water were mixed to prepare a sample having a predetermined CNF/methyl cellulose/water ratio.

[Comparative Example 2]

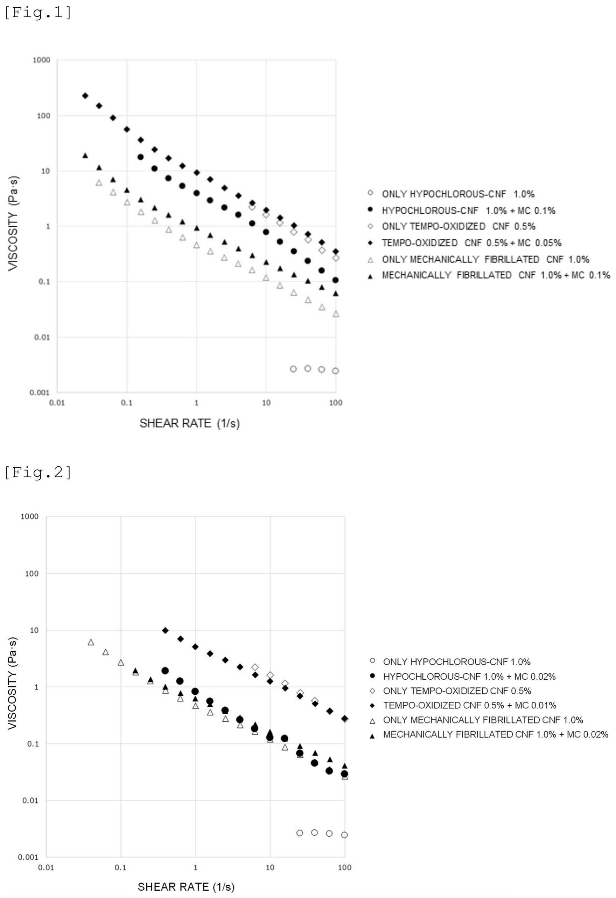

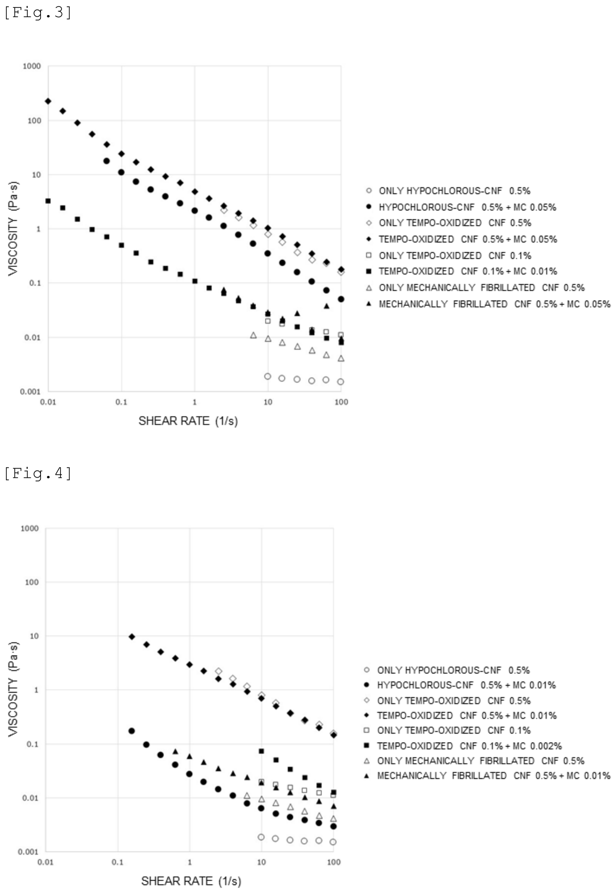

The aqueous nanocellulose dispersion (solid content of 2.0 mass%) obtained by mechanical fibrillation, which was produced in Comparative Production Example 2, a 1 mass% aqueous solution of methyl cellulose, and distilled water were mixed to prepare a sample having a predetermined CNF/methyl cellulose/water ratio.
Measurement results of various samples in Example 1, Comparative Example 1, and Comparative Example 2 are shown in Figs. 1 to 4. Note that % of each component in the figures represents mass% of each component with respect to the total component amount of each sample.

[Example 2]

The oxidized cellulose obtained in Production Example 1 was used as an aqueous dispersion (solid content of about 10 mass%), and distilled water was added thereto to dilute the oxidized cellulose to a solid content of 5 mass%. In this state, the mixture was stirred at 10000 rpm for 60 minutes using a homomixer (TOKUSHU KIKA ROBO MICS) to fibrillate the oxidized cellulose into CNF (pre-fibrillation).
Separately, an acrylic emulsion having a solid content of 18 mass% and a 1 mass% aqueous solution of methyl cellulose were prepared. A 5 mass% CNF aqueous dispersion, an acrylic emulsion, a 1 mass% aqueous solution of methyl cellulose, and distilled water were mixed to prepare Sample 1 having a predetermined CNF/emulsion/methyl cellulose ratio.
The viscosity was measured by the same viscosity measurement method as in Example 1.

[Comparative Example 3]

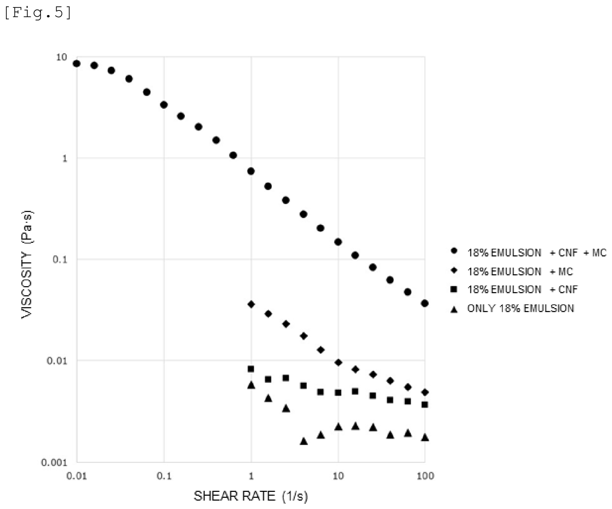

From the sample of Example 2, Sample 2 from which CNF was removed, Sample 3 from which methyl cellulose was removed, and Sample 4 from which CNF and methyl cellulose were removed were prepared.
The viscosity was measured by the same viscosity measurement method as in Example 1.
The compositions of various samples in Example 2 and Comparative Example 3 are shown in Table 1, and the measurement results are shown in Fig. 5.

[Table 1]

|  | Sample 1 | Sample 2 | Sample 3 | Sample 4 |
|---|---|---|---|---|
| Acrylic emulsion | 18.2 | 18.2 | 18.2 | 18.2 |
| water | 80.8 | 81.71 | 80.89 | 81.8 |
| CNF | 0.91 | 0 | 0.91 | 0 |
| Methyl cellulose | 0.091 | 0.091 | 0 | 0 |

The values for components other than water are represented as mass% of solid content

[Example 3]

The oxidized cellulose obtained in Production Example 1 was used as an aqueous dispersion (solid content of about 10 mass%), and distilled water was added thereto to dilute the oxidized cellulose to a solid content of 5 mass%. In this state, the mixture was stirred at 10000 rpm for 60 minutes using a homomixer (TOKUSHU KIKA ROBO MICS) to fibrillate the oxidized cellulose into CNF.
A sample obtained by mixing CNF, methyl cellulose, and water at a predetermined ratio was adjusted.
The viscosity was measured by the same viscosity measurement method as in Example 1.

[Example 4]

A sample using guar gum (GG) instead of the methyl cellulose of Example 3 was adjusted.

The viscosity was measured by the same viscosity measurement method as in Example 1.

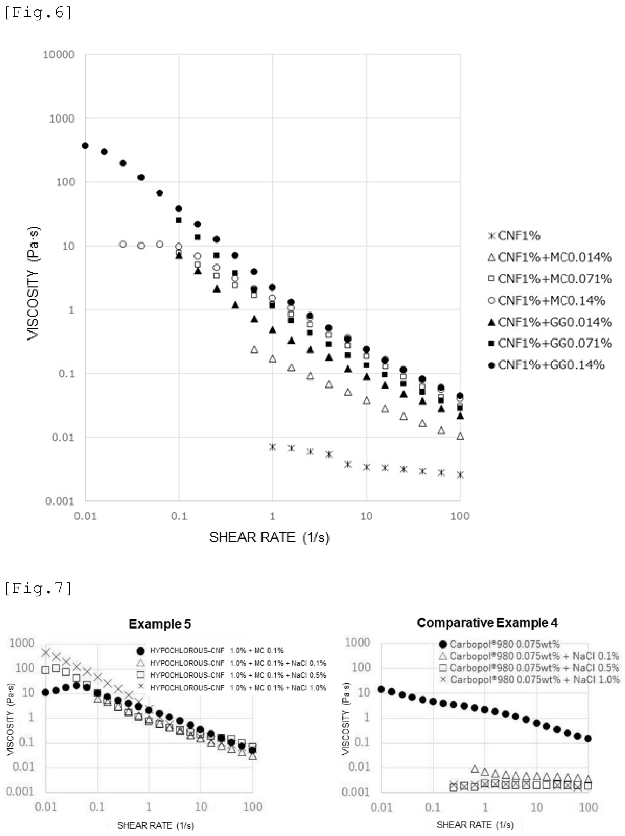

Measurement results of various samples in Examples 3 and 4 are shown in Fig. 6. Note that % of each component in the figures represents mass% of each component with respect to the total component amount of each sample.

[Example 5]

The oxidized cellulose obtained in Production Example 1 was used as an aqueous dispersion (solid content of about 10 mass%), and distilled water was added thereto to dilute the oxidized cellulose to a solid content of 7.5 mass%. In this state, the mixture was stirred at 10000 rpm for 60 minutes using a homomixer (TOKUSHU KIKA ROBO MICS) to fibrillate the oxidized cellulose into CNF.

Separately, an aqueous solution having a concentration of 1 mass% of methyl cellulose (MC) was prepared. In addition, an aqueous solution having a concentration of 12.5 mass% of sodium chloride (NaCl) and distilled water were prepared. A CNF aqueous dispersion having a solid content of 7.5 mass%, a 1 mass% aqueous solution of methyl cellulose, a 12.5 mass% aqueous solution of sodium chloride, and distilled water were mixed to prepare a sample in which a ratio of CNF/methyl cellulose/sodium chloride to the total amount of the composition was 1.0 mass%/0.1 mass%/0 to 1.0 mass%.

The viscosity of each sample was measured by the same viscosity measurement method as in Example 1.

[Comparative Example 4]

A 12.5 mass% aqueous solution of sodium chloride and distilled water were mixed using Carbopol (registered trademark) 980 to prepare a sample in which a ratio of Carbopol (registered trademark)/sodium chloride with respect to the total amount of the composition was 0.075 mass%/0 to 1.0 mass%.

The viscosity of each sample was measured by the same viscosity measurement method as in Example 1.

[Comparative Example 5]

A 12.5 mass% aqueous solution of sodium chloride and distilled water were mixed using Carbopol (registered trademark) 980 to prepare a sample in which a ratio of Carbopol (registered trademark)/sodium chloride with respect to the total amount of the composition was 1.0 mass%/0 to 1.0 mass%.

The viscosity of each sample was measured by the same viscosity measurement method as in Example 1.

The results of viscosity measurement of Example 5 (CNF and MC) and Comparative Example 4 (Carbopol (registered trademark) 980) are shown in Fig. 7. The sample containing no salt in Example 5 and the sample containing no salt in Comparative Example 4 had substantially the same viscosity. In Comparative Example 4, the viscosity was significantly decreased in a case where a salt was added, but in Example 5, a decrease in viscosity was minimized even when a salt was added.

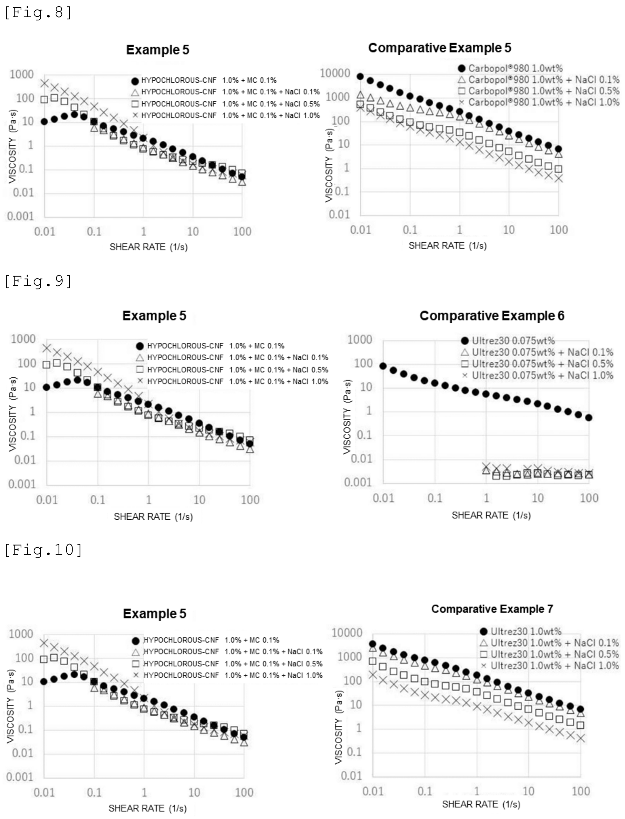

The results of viscosity measurement of Example 5 (CNF and MC) and Comparative Example 5 (Carbopol (registered trademark) 980) are shown in Fig. 8. The amount of CNF in Example 5 and the amount of Carbopol (registered trademark) in Comparative Example 5 were the same. In Comparative Example 5, the viscosity was decreased in a case where a salt was added, but in Example 5, a decrease in viscosity was minimized even when a salt was added.

[Comparative Example 6]

A 12.5 mass% aqueous solution of sodium chloride and distilled water were mixed using Carbopol (registered trademark) Ultrez 30 (hereinafter, also simply described as Ultrez 30) to prepare a sample in which a ratio of Ultrez 30/sodium chloride with respect to the total amount of the composition was 0.075 mass%/0 to 1.0 mass%.

The viscosity of each sample was measured by the same viscosity measurement method as in Example 1.

[Comparative Example 7]

A 12.5 mass% aqueous solution of sodium chloride and distilled water were mixed using Ultrez 30 to prepare a sample in which a ratio of Ultrez 30/sodium chloride with respect to the total amount of the composition was 1.0 mass%/0 to 1.0 mass%.

The viscosity of each sample was measured by the same viscosity measurement method as in Example 1.

The results of viscosity measurement of Example 5 (CNF and MC) and Comparative Example 6 (Ultrez 30) are shown in Fig. 9. The sample containing no salt in Example 5 and the sample containing no salt in Comparative Example 6 had substantially the same viscosity. In Comparative Example 6, the viscosity was significantly decreased in a case where a salt was added, but in Example 5, a decrease in viscosity was minimized even when a salt was added.

The results of viscosity measurement of Example 5 (CNF and MC) and Comparative Example 7 (Ultrez 30) are shown in Fig. 10. The amount of CNF in Example 5 and the amount of Ultrez 30 in Comparative Example 7 were the same. In Comparative Example 7, the viscosity was decreased in a case where a salt was added, but in Example 5, a decrease in viscosity was minimized even when a salt was added.

[Example 6]

The oxidized cellulose obtained in Production Example 1 was used as an aqueous dispersion (solid content of about 10 mass%), and distilled water was added thereto to dilute the oxidized cellulose to a solid content of 7.5 mass%. In this state, the mixture was stirred at 10000 rpm for 60 minutes using a homomixer (TOKUSHU KIKA ROBO MICS) to fibrillate the oxidized cellulose into CNF.

Separately, an aqueous solution having a concentration of 1 mass% of methyl cellulose (MC) and distilled water were prepared. A CNF aqueous dispersion having a solid content of 7.5 mass%, a 1 mass% aqueous solution of methyl cellulose, and distilled water were mixed to prepare a sample having a ratio of CNF 1 mass%/methyl cellulose 0.1 mass% to the total amount of the composition.

The pH of the sample was adjusted using a 0.5 M hydrochloric acid or a 0.5 M sodium hydroxide aqueous solution. A 0.5 M hydrochloric acid was used for adjustment to the acidic side, and a 0.5 M aqueous sodium hydroxide solution was used for adjustment to the alkaline side, and an electrode of a pH meter (HORIBA D-51 pH METER) was immersed in a screw tube containing the above-described sample, and the reagent was added dropwise while confirming the value.

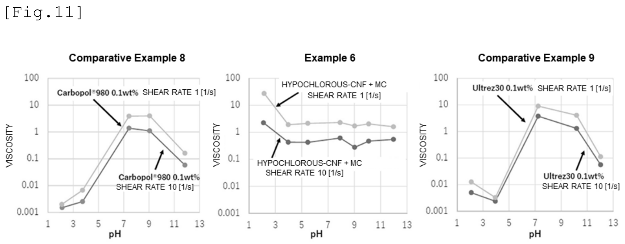

The viscosity was measured by the same viscosity measurement method as in Example 1 at shear rates of 1 [1/s] and 10 [1/s]. The results are shown in Fig. 11.

[Comparative Example 8]

Carbopol (registered trademark) 980 and distilled water were mixed to prepare a sample in which Carbopol (registered trademark) has a ratio of 0.1 mass% to the total composition amount. Viscosity measurement was performed in the same manner as in Example 6 except that this sample was used. The results are shown in Fig. 11.

[Comparative Example 9]

Carbopol (registered trademark) Ultrez 30 and distilled water were mixed to prepare a sample in which Ultrez 30 has a ratio of 0.1 mass% to the total composition amount. Viscosity measurement was performed in the same manner as in Example 6 except that this sample was used. The results are shown in Fig. 11.

As shown in Fig. 11, the composition of Example 6 (CNF and MC) tends to be able to minimize the viscosity decrease even when the pH is changed from 7. On the other hand, in the composition of Comparative Example 8 (Carbopol (registered trademark) 980) or the composition of Comparative Example 9 (Ultrez 30), the viscosity tends to decrease when the pH is changed from 7.

Therefore, a dispersion containing the oxidized cellulose that is an oxide obtained by oxidizing a cellulose raw-material with hypochlorous acid or a salt thereof, and/or nanocellulose that is a fibrillated product of the oxidized cellulose, and a thickener can increase the viscosity, and can minimize a decrease in the viscosity due to a pH change.

**Claims**

1. A composition comprising: a cellulose; and a thickener, wherein
   the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

2. A composition comprising: a cellulose; and a thickener, wherein
   the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

3. The composition according to claim 1 or 2, wherein
the thickener is a thickening polysaccharide.

4. The composition according to claim 1 or 2, wherein
the thickener has a hydrophobic region capable of hydrophobic interaction with the oxidized cellulose and/or the nanocellulose.

5. The composition according to claim 1 or 2, wherein
an amount of the thickener is 0.1 to 40 mass% with respect to a mass (solid content) of the oxidized cellulose and/or the nanocellulose.

6. The composition according to claim 1 or 2, further comprising a salt.

7. The composition according to claim 6, wherein
an amount of the salt is 0.01 to 10 mass% with respect to the composition.

8. The composition according to claim 1 or 2, further comprising a pH adjuster.

9. The composition according to claim 8, wherein
the composition has a pH in a range of 0 or more and 14.0 or less.

10. The composition according to claim 8, wherein
the pH adjuster comprises an acid or a base.

11. The composition according to claim 1 or 2, wherein
the cellulose comprises the nanocellulose.

12. The composition according to claim 1 or 2, wherein
the composition is an aqueous composition.

13. The composition according to claim 12, wherein
the aqueous composition has a viscosity of 0.01 to 1000 Pa·s.

14. The composition according to claim 12, wherein
the aqueous composition has a viscosity of 10 to $1 \times 10^6$% higher than a viscosity of a control composition obtained by removing the thickener from the aqueous composition.

15. A composition comprising: a cellulose; and a thickener, wherein

the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound, and
the composition has a pH in a range of 0 or more and 14.0 or less.

16. A composition comprising: a cellulose; and a thickener, wherein

the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose, and
the composition has a pH in a range of 0 or more and 14.0 or less.

17. The composition according to any one of claims 1, 2, 15, and 16, wherein
the composition is a cosmetic product.

18. A method for producing a composition comprising:

a step of mixing a cellulose and a thickener, wherein
the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

19. A method for producing a composition comprising:

a step of mixing a cellulose and a thickener, wherein
the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

20. A method for modifying a viscosity of an aqueous composition comprising a cellulose, the method comprising:

a step of mixing the aqueous composition and a thickener, wherein
the cellulose comprises an oxidized cellulose that is an oxide obtained by oxidizing a cellulose raw-material with a hypochlorous acid or a salt thereof and/or a nanocellulose that is a fibrillated product of the oxidized cellulose, and is substantially free of an N-oxyl compound.

21. A method for modifying a viscosity of an aqueous composition comprising a cellulose, the method comprising:

a step of mixing the aqueous composition and a thickener, wherein
the cellulose comprises an oxidized cellulose that has a structure in which hydroxyl groups at position 2 and position 3 of a glucopyranose ring of the cellulose are oxidized and dicarboxy groups are introduced, and/or a nanocellulose that is a fibrillated product of the oxidized cellulose.

22. The method according to claim 20 or 21, wherein
the viscosity of the aqueous composition is increased by 10 to $1 \times 10^6$% by mixing the aqueous composition and the thickener.

[Fig.1]

- ○ ONLY HYPOCHLOROUS-CNF 1.0%
- ● HYPOCHLOROUS-CNF 1.0% + MC 0.1%
- ◇ ONLY TEMPO-OXIDIZED CNF 0.5%
- ◆ TEMPO-OXIDIZED CNF 0.5% + MC 0.05%
- △ ONLY MECHANICALLY FIBRILLATED CNF 1.0%
- ▲ MECHANICALLY FIBRILLATED CNF 1.0% + MC 0.1%

[Fig.2]

- ○ ONLY HYPOCHLOROUS-CNF 1.0%
- ● HYPOCHLOROUS-CNF 1.0% + MC 0.02%
- ◇ ONLY TEMPO-OXIDIZED CNF 0.5%
- ◆ TEMPO-OXIDIZED CNF 0.5% + MC 0.01%
- △ ONLY MECHANICALLY FIBRILLATED CNF 1.0%
- ▲ MECHANICALLY FIBRILLATED CNF 1.0% + MC 0.02%

[Fig.3]

[Fig.4]

[Fig.5]

[Fig.6]

[Fig.7]

[Fig.8]

[Fig.9]

[Fig.10]

[Fig.11]

## INTERNATIONAL SEARCH REPORT

| International application No. |
|---|
| **PCT/JP2024/020495** |

**A. CLASSIFICATION OF SUBJECT MATTER**

*C08B 15/02*(2006.01)i; *A61K 8/73*(2006.01)i; *C08B 15/04*(2006.01)i; *C08L 1/04*(2006.01)i; *C09K 3/00*(2006.01)i
FI: C08B15/02; A61K8/73; C08L1/04 ZNM; C08B15/04; C09K3/00 103G

According to International Patent Classification (IPC) or to both national classification and IPC

**B. FIELDS SEARCHED**

Minimum documentation searched (classification system followed by classification symbols)

C08B15/02; A61K8/73; C08B15/04; C08L1/04; C09K3/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2012-126788 A (DKS CO. LTD.) 05 July 2012 (2012-07-05) claims, paragraphs [0018], [0026], [0043], [0062], [0065], examples 1-12 | 1, 3-15, 17-18, 20, 22 |
| Y | | 1-22 |
| Y | WO 2022/009980 A1 (TOAGOSEI CO., LTD.) 13 January 2022 (2022-01-13) claims, paragraphs [0016], [0045], examples | 1-22 |
| Y | WO 2018/030392 A1 (NIPPON PAPER INDUSTRIES CO., LTD.) 15 February 2018 (2018-02-15) paragraphs [0002], [0041] | 1-22 |
| Y | JP 2017-36217 A (OJI HOLDINGS CORPORATION) 16 February 2017 (2017-02-16) claims, paragraph [0002], examples | 1-22 |
| A | JP 2020-33398 A (OJI HOLDINGS CORPORATION) 05 March 2020 (2020-03-05) claims, examples | 1-22 |

☑ Further documents are listed in the continuation of Box C.    ☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **26 July 2024** | **13 August 2024** |

| Name and mailing address of the ISA/JP | Authorized officer |
|---|---|
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (July 2022)

**INTERNATIONAL SEARCH REPORT**

International application No.

**PCT/JP2024/020495**

| C. | DOCUMENTS CONSIDERED TO BE RELEVANT | |
|---|---|---|
| Category* | Citation of document, with indication, where appropriate, of the relevant passages | Relevant to claim No. |
| A | JP 2023-13727 A (TOAGOSEI CO., LTD.) 26 January 2023 (2023-01-26)<br>claims, examples | 1-22 |

Form PCT/ISA/210 (second sheet) (July 2022)

## INTERNATIONAL SEARCH REPORT
### Information on patent family members

International application No.

**PCT/JP2024/020495**

| Patent document cited in search report | | | Publication date (day/month/year) | Patent family member(s) | Publication date (day/month/year) |
|---|---|---|---|---|---|
| JP | 2012-126788 | A | 05 July 2012 | (Family: none) | |
| WO | 2022/009980 | A1 | 13 January 2022 | US 2023/0250198 A1 claims, paragraphs [0021], [0062], examples<br>EP 4180461 A1<br>CN 115916845 A | |
| WO | 2018/030392 | A1 | 15 February 2018 | US 2019/0353634 A1 paragraphs [0002], [0065]<br>EP 4179880 A1<br>KR 10-2019-0008282 A<br>CN 109219357 A | |
| JP | 2017-36217 | A | 16 February 2017 | US 2018/0296446 A1 claims, paragraph [0002], examples<br>EP 3332763 A1<br>KR 10-2018-0043793 A<br>CN 108024942 A | |
| JP | 2020-33398 | A | 05 March 2020 | (Family: none) | |
| JP | 2023-13727 | A | 26 January 2023 | (Family: none) | |

Form PCT/ISA/210 (patent family annex) (July 2022)

REFERENCES CITED IN THE DESCRIPTION

**Patent documents cited in the description**

- WO 2018230354 A
- WO 2020027307 A
- WO 2022009979 A
- WO 2022009980 A

**Non-patent literature cited in the description**

- *Sustainable Chem. Eng.*, 2020, vol. 8 (48), 17800-17806
- *Angew. Chem. Int. Ed.*, 2021, vol. 60, 24630-24636